# EUROPEAN PATENT APPLICATION

(11) **EP 2 441 836 A1**
(43) Date of publication of application: **18.04.2012**
(21) Application number: 10187564.9
(22) Date of filing: 14.10.2010
(51) Int. Cl.: C12N 15/113, A61K 31/7088, A61P 35/00

(54) **Compound inhibiting TP53INP1 activity for the treatment of prostate cancer, and its use in screening and diagnostic methods**

(71) Applicant: Assistance Publique Hôpitaux de Marseille, 13354 Marseille (FR)
(72) Inventor: Dusetti, Nelson, 13008, MARSEILLE (FR); Rocchi, Palma, 13008, MARSEILLE (FR); Garcia, Stéphane, 13100, AIX-EN-PROVENCE (FR); Giusiano, Sophie, 13380, PLAN-DE-CUQUES (FR); Iovanna, Juan, 13008, MARSEILLE (FR)
(74) Representative: Novagraaf Technologies

(57) **Abstract**

The present invention refers to a compound inhibiting TP53INP1 activity for use in treatment of prostate cancer, a method for in vitro diagnosing a prostate cancer comprising the step of determining the level of expression of β transcript of TP53INP1 gene in a patient-derived biological sample comprising a prostate cell, and to a method of screening for compounds modulating the TP53INP1 activity.

SEQUENCE LISTING

<110>  AP-HM

<120>  Compound inhibiting TP53INP1 activity for the treatment of
       prostate cancer, and its use in screening and diagnostic methods

<130>  BNT207556EP00

<160>  11

<170>  PatentIn version 3.5

<210>  1
<211>  240
<212>  PRT
<213>  Homo sapiens

<400>  1

Met Phe Gln Arg Leu Asn Lys Met Phe Val Gly Glu Val Ser Ser Ser
1               5                   10                  15

Ser Asn Gln Glu Pro Glu Phe Asn Glu Lys Glu Asp Asp Glu Trp Ile
            20                  25                  30

Leu Val Asp Phe Ile Asp Thr Cys Thr Gly Phe Ser Ala Glu Glu Glu
        35                  40                  45

Glu Glu Glu Glu Asp Ile Ser Glu Glu Ser Pro Thr Glu His Pro Ser
    50                  55                  60

Val Phe Ser Cys Leu Pro Ala Ser Leu Glu Cys Leu Ala Asp Thr Ser
65                  70                  75                  80

Asp Ser Cys Phe Leu Gln Phe Glu Ser Cys Pro Met Glu Glu Ser Trp
                85                  90                  95

Phe Ile Thr Pro Pro Pro Cys Phe Thr Ala Gly Gly Leu Thr Thr Ile
            100                 105                 110

Lys Val Glu Thr Ser Pro Met Glu Asn Leu Leu Ile Glu His Pro Ser
        115                 120                 125

Met Ser Val Tyr Ala Val His Asn Ser Cys Pro Gly Leu Ser Glu Ala
    130                 135                 140

Thr Arg Gly Thr Asp Glu Leu His Ser Pro Ser Ser Pro Arg Val Glu
145                 150                 155                 160

Ala Gln Asn Glu Met Gly Gln His Ile His Cys Tyr Val Ala Ala Leu
                165                 170                 175

Ala Ala His Thr Thr Phe Leu Glu Gln Pro Lys Ser Phe Arg Pro Ser
            180                 185                 190

Gln Trp Ile Lys Glu His Ser Glu Arg Gln Pro Leu Asn Arg Asn Ser

195                 200                 205

Leu Arg Arg Gln Asn Leu Thr Arg Asp Cys His Pro Arg Gln Val Lys
    210                 215                 220

His Asn Gly Trp Val Val His Gln Pro Cys Pro Arg Gln Tyr Asn Tyr
225                 230                 235                 240

<210>  2
<211>  164
<212>  PRT
<213>  Homo sapiens

<400>  2

Met Phe Gln Arg Leu Asn Lys Met Phe Val Gly Glu Val Ser Ser Ser
1               5                   10                  15

Ser Asn Gln Glu Pro Glu Phe Asn Glu Lys Glu Asp Asp Glu Trp Ile
            20                  25                  30

Leu Val Asp Phe Ile Asp Thr Cys Thr Gly Phe Ser Ala Glu Glu Glu
        35                  40                  45

Glu Glu Glu Glu Asp Ile Ser Glu Glu Ser Pro Thr Glu His Pro Ser
    50                  55                  60

Val Phe Ser Cys Leu Pro Ala Ser Leu Glu Cys Leu Ala Asp Thr Ser
65                  70                  75                  80

Asp Ser Cys Phe Leu Gln Phe Glu Ser Cys Pro Met Glu Glu Ser Trp
                85                  90                  95

Phe Ile Thr Pro Pro Pro Cys Phe Thr Ala Gly Gly Leu Thr Thr Ile
            100                 105                 110

Lys Val Glu Thr Ser Pro Met Glu Asn Leu Leu Ile Glu His Pro Ser
        115                 120                 125

Met Ser Val Tyr Ala Val His Asn Ser Cys Pro Gly Leu Ser Glu Ala
    130                 135                 140

Thr Arg Gly Thr Asp Glu Leu His Ser Pro Ser Ser Pro Arg Ala Arg
145                 150                 155                 160

Lys Ser Cys Leu

<210>  3
<211>  5628
<212>  DNA
<213>  Homo sapiens

<400>  3
cgatcagctg ttgcgagcgc tgcacaacaa caaaaggact tggactggcc ggcctgggcg        60

cagcgacccg agggctggag ccggccccgc gcctgccgtc tgggtacctg aacgaggtgc 120
agcgcagccc ggccccaccg cagctacctc agcagtcccg ccccgcccgc gtccttcccc 180
gccgagccgg cggccgctcc cttccccgcg cagccccgca cggcccgggc ccacgtacaa 240
tgactcttct tgcttttcac ctaagttgaa taagcaccct gtgcacttta atctcctgtc 300
ggtaccattg ggccaactaa agacaaggtt ttgaaatctc agctataaaa gacatccagc 360
caaactctca gtcttgcctt aacaatgttc cagaggctga ataaaatgtt tgtgggtgaa 420
gtcagttctt cctccaacca agaaccagaa ttcaatgaga aagaagatga tgaatggatt 480
cttgttgact tcatagatac ttgcactggt ttctcagcag aagaagaaga agaagaggag 540
gacatcagtg aagagtcacc tactgagcac ccttcagtct tttcctgttt accggcatct 600
cttgagtgct tggctgatac aagtgattcc tgctttctcc agtttgagtc atgtccaatg 660
gaggagagct ggtttatcac cccaccccca tgttttactg caggtggatt aaccactatc 720
aaggtggaaa caagtcctat ggaaaacctt ctcattgaac atcccagcat gtctgtctat 780
gctgtgcata actcctgccc tggtctcagt gaggccaccc gtgggactga tgaattacat 840
agcccaagta gtcccagagt ggaagctcaa aatgaaatgg ggcagcatat tcattgttat 900
gttgcagctc ttgctgctca tacaactttt ctggaacaac ccaagagctt tcgcccttcc 960
cagtggataa aagaacacag tgaaagacag cctcttaaca gaaatagcct tcgtcgccaa 1020
aatcttacca gggattgcca ccctcggcaa gtcaagcaca atggctgggt tgttcatcag 1080
ccctgcccgc gtcagtacaa ttactaatag tttcaagttt tgttggttgg tttctcttgg 1140
tttgtgctta catgtatgga tgtgtgtata tgtacagtga aaatgttgtc tctttacaac 1200
caattgataa ccaatcacat agttttatca gtgtatttag acactatctt gaaaatcaga 1260
tttatatgct gtgtatcaca taatgccttg cctttaacat ttactttttt tgtacacttt 1320
ttcagattat ttctggaaac atatcaatat aattacagtg tttgggggtg tctttaaata 1380
tattaggtta tacattagtc agcattttaa agacatttct tcccaagtac gagaataggc 1440
atctttcatt ttcattttat tttgtattac ttaatctttt aagcaagcaa aaatttattc 1500
tcagggtcag ctgtacactt tattgaccag tacttgataa tctctctgta tatgatgaat 1560
acatttttac acactaacat tagcattaac aggtgatagt tgccatggat ataatggaat 1620
tatggctgga ctttcttttg aaagaaaact tgatgtattc tgtgtgtatg gtttttcccc 1680
agattagtca tacagttcat ttggaattca ggtacattaa gctttagtga agagtgcatg 1740
cagtaattcc aatgtgactg catgacgtgg tacagacatt acaggtgttg tagacagagg 1800
cacttgtctc gtgcagaggg attaaattag acctgtgaga ttatatttgg aaaaattcat 1860
gtctgtaact aacccattag tgcagtattt aatttgttac tattccttcc cgccaattct 1920
gtccactcct cacctcgcat cagctataaa tttggaagta cttgtccagg cactcaagtg 1980
acttcatatt tctctctgcc catgggaaaa gagataggct ttatatttcc acagagtgaa 2040
aaatcctctg tcatggagcc tgtcctgcca agtggcaaga gtgtggggac tgtctggtga 2100

tgatgtcttt catggcatct gagtgaagag tgacaggttg gctcaacttt tttctttttt 2160
tttttttaat tgccttgtat tgtaagtatt cttccctgca gtccaagtga cttttcattt 2220
tttgttttaa cttcaggcaa aatctttaac cactctggcc tctgtttccc ccaccaacgg 2280
ggagcagtga catttacctc cctcacagag tcactgtgag gattctatac tgatttgaag 2340
tggagctgtt cagaactgaa ccttgtagga aattccaagg gcctttctac tgaatctggt 2400
gatggggtgg ggccgtggca ctttctctgc cacagctgtt cttcacagtg ttggtgctaa 2460
tgaggccagg gtgcagggtt cgattcacac gtaggccagt taacttagag aaaatctatt 2520
tccttacctc tagccagtca cttccttttt ccgcagttgt gatgggtttt gctgagccat 2580
ccactctgac tgatttcctc tgaagtaaac atatttacaa tccaaagcaa ttctactgac 2640
agaagtgttg ccttcataat caaacagctt gtttttccat ctcctctgca accctaatta 2700
aatgagtaca ggtctacaaa atgttttcaa ggagaaaagc agcatatcct taagtgaagt 2760
attatatttt tcaataaccc tgtagtggct tgatgcaggg aaccctgggg gactttcagc 2820
gaagagctgt gctcttttct gactagatta gagcgtttgg agtggaagac gtcaaatgtg 2880
tagtgagatg gaggttttac attgttcttc tactggctgt gatgaagtgc cagaatgtct 2940
ctttagaaca agagttagat tccccctttc tccttattgc cccttccgtt ttgacttccc 3000
ctttatttat ttgttgtcta attaggggcc aagtctgtaa agttttgtca aagtgagtta 3060
gaagttgttt tctcttacta tttgtgttta ccagagttgg gagataagat agtttccatg 3120
aaggtgtgta tgttttatac gatgtttgtt atagggccat gcattggtaa cttgaaaata 3180
gaccagctta atgtcttcag gatgtaaaac tctgaataca cggcgtctct tttcatacaa 3240
ttgcatgtaa gttgttagta cctcacaagc tacagaagtt cagccatgag attttgtttg 3300
gcaacatgaa cagatttgtg tataactgca atggcctttt tttccagatt tccttattga 3360
ctttttgttt gccttacctg gggctagttt tttatgcttt gtacctagaa aacaaaaaat 3420
tacattcgtt gggctttttt tcaaggttgg gattaccaca ccacctggaa tatcatactg 3480
tggtttctgc ctaaaattgg cacatgtaag tattgaagaa aatggttata taattcagtt 3540
gaaactcttg gttattagat gttaggcatc tcctgtatgt aagacacaag gccaaccaca 3600
acacagaacg atgttgacct gttaagtatt ctctgaaaca tggccaaaat gcattttatg 3660
agcttttttt tttgctattg taaatattag tggtttacaa tgcgctttag acatatttct 3720
ttaaaatgca agcagtgaga aataagacct ctctgaatta gtagctctaa actgttaaca 3780
tagaatgtta cttggaaaaa gtctggaata tgtggtgtac acaagcagtg cttcgtgaat 3840
gagtttctta gcttttatag tgcgccatgt ttctcaaagt ttgtttttgt tgacaaaaca 3900
ttttataata tatatcttat gtttattttt tttctcaact aattgtgtac tgcactgtaa 3960
ggtgaaaatt agccatccat tatttatctt ctgtggcaat gcatttatat ggttgattgg 4020
gtggggaatt ttttgcagaa agatgcaaag tgattgggtt ttcgacttcc tatcgcaggg 4080
agcttttaag aaatattaat ttcctataca tttttccaat ccccatgcaa actgttcctg 4140
tttacatacc ttctctgttg tatcagtact ttgagtgaga agacagttta tttaaaactt 4200

gagcaggctg ttcagcattg tttctgcttc tgaaatctgt atagtacact ggtttgtaat 4260

cattatgtct tcattgaaat ccttgctact tctcttcctc ctcaatgaaa tacattatat 4320

attatcttta tgtactctta agaaaaacga gcaaggaaga gtatcttcat tattctcatt 4380

ttctctgagt tggaaacaaa aacatgaagg actccaacta gaagacagat atttacattt 4440

aaatagatta gtgggaaaac tttaagagtt tccacatatt agttttcatt ttttgagtca 4500

agagactgct ccttgtactg ggagacacta gtagtatatg tttgtaatgt tactttaaaa 4560

ttatcttttt attttataag gcccataaat actggttaaa ctctgttaaa agtgggcctt 4620

ctatcttgga tggtttcact gccatcagcc atgctgatat attagaaatg gcatccctat 4680

ctacttactt taatgcttaa aattatacat aaaatgcttt atttagaaaa cctacatgat 4740

acagtggtgt cagccttgcc atgtatcagt ttcacttgaa atttgagacc aattaaattt 4800

caactgttta gggtggagaa agaggtactg gaaaacatgc agatgaggat atcttttatg 4860

tgcaacagta tcctttgcat gggaggagag ttactcttga aaggcaggca gcttaagtgg 4920

acaatgtttt gtatatagtt gagaatttta cgacactttt aaaaattgtg taattgttaa 4980

atgtccagtt ttgctctgtt ttgcctgaag ttttagtatt tgttttctag gtggacctct 5040

gaaaaccaaa ccagtacctg gggaggttag atgtgtgttt caggcttgga gtgtatgagt 5100

ggttttgctt gtattttcct ccagagattt tgaactttaa taattgcgtg tgtttttttt 5160

ttttttaagt ggctttgttt ttttttctca agtaaaattg tgaacatatt tcctttatag 5220

gggcagggca tgagttaggg agactgaaga gtattgtaga ctgtacatgt gccttcttaa 5280

tgtgtttctc gacacatttt ttttcagtaa cttgaaaatt caaaagggac atttggttag 5340

gttactgtac atcaatctat gcataaatgg cagcttgttt tcttgagcca cggtctaaat 5400

tttgttttta tagaaatttt ttatactgat tggttcatag atggtcagtt ttgtacacag 5460

actgaacaat acagcacttt gccaaaaatg agtgtagcat tgtttaaaca ttgtgtgtta 5520

acacctgttc tttgtaattg ggttgtggtg cattttgcac tacctggagt tacagttttc 5580

aatctgtcag taaataaagt gtcctttaac ttcaaaaaaa aaaaaaaa 5628

<210> 4
<211> 5666
<212> DNA
<213> Homo sapiens

<400> 4
cgatcagctg ttgcgagcgc tgcacaacaa caaaaggact tggactggcc ggcctgggcg 60

cagcgacccg agggctggag ccggccccgc gcctgccgtc tgggtacctg aacgaggtgc 120

agcgcagccc ggccccaccg cagctacctc agcagtcccg ccccgcccgc gtccttcccc 180

gccgagccgg cggccgctcc cttccccgcg cagccccgca cggcccgggc ccacgtacaa 240

tgactcttct tgcttttcac ctaagttgaa taagcaccct gtgcacttta atctcctgtc 300

ggtaccattg ggccaactaa agacaaggtt ttgaaatctc agctataaaa gacatccagc 360

caaactctca gtcttgcctt aacaatgttc cagaggctga ataaaatgtt tgtgggtgaa 420

gtcagttctt cctccaacca agaaccagaa ttcaatgaga aagaagatga tgaatggatt 480
cttgttgact tcatagatac ttgcactggt ttctcagcag aagaagaaga agaagaggag 540
gacatcagtg aagagtcacc tactgagcac ccttcagtct tttcctgttt accggcatct 600
cttgagtgct tggctgatac aagtgattcc tgctttctcc agtttgagtc atgtccaatg 660
gaggagagct ggtttatcac cccaccccca tgttttactg caggtggatt aaccactatc 720
aaggtggaaa caagtcctat ggaaaacctt ctcattgaac atcccagcat gtctgtctat 780
gctgtgcata actcctgccc tggtctcagt gaggccaccc gtgggactga tgaattacat 840
agcccaagta gtcccagggc caggaaaagc tgcttataag actcacgggc acagaagtgg 900
aagctcaaaa tgaaatgggg cagcatattc attgttatgt tgcagctctt gctgctcata 960
caacttttct ggaacaaccc aagagctttc gcccttccca gtggataaaa gaacacagtg 1020
aaagacagcc tcttaacaga aatagccttc gtcgccaaaa tcttaccagg gattgccacc 1080
ctcggcaagt caagcacaat ggctgggttg ttcatcagcc ctgcccgcgt cagtacaatt 1140
actaatagtt tcaagttttg ttggttggtt tctcttggtt tgtgcttaca tgtatggatg 1200
tgtgtatatg tacagtgaaa atgttgtctc tttacaacca attgataacc aatcacatag 1260
ttttatcagt gtatttagac actatcttga aaatcagatt tatatgctgt gtatcacata 1320
atgccttgcc tttaacattt actttttttg tacacttttt cagattattt ctggaaacat 1380
atcaatataa ttacagtgtt tgggggtgtc tttaaatata ttaggttata cattagtcag 1440
cattttaaag acatttcttc ccaagtacga gaataggcat ctttcatttt cattttattt 1500
tgtattactt aatcttttaa gcaagcaaaa atttattctc agggtcagct gtacacttta 1560
ttgaccagta cttgataatc tctctgtata tgatgaatac atttttacac actaacatta 1620
gcattaacag gtgatagttg ccatggatat aatggaatta tggctggact ttcttttgaa 1680
agaaaacttg atgtattctg tgtgtatggt ttttccccag attagtcata cagttcattt 1740
ggaattcagg tacattaagc tttagtgaag agtgcatgca gtaattccaa tgtgactgca 1800
tgacgtggta cagacattac aggtgttgta gacagaggca cttgtctcgt gcagagggat 1860
taaattagac ctgtgagatt atatttggaa aaattcatgt ctgtaactaa cccattagtg 1920
cagtatttaa tttgttacta ttccttcccg ccaattctgt ccactcctca cctcgcatca 1980
gctataaatt tggaagtact tgtccaggca ctcaagtgac ttcatatttc tctctgccca 2040
tgggaaaaga gataggcttt atatttccac agagtgaaaa atcctctgtc atggagcctg 2100
tcctgccaag tggcaagagt gtggggactg tctggtgatg atgtctttca tggcatctga 2160
gtgaagagtg acaggttggc tcaacttttt tctttttttt tttttaattg ccttgtattg 2220
taagtattct tccctgcagt ccaagtgact tttcattttt tgttttaact tcaggcaaaa 2280
tctttaacca ctctggcctc tgtttccccc accaacgggg agcagtgaca tttacctccc 2340
tcacagagtc actgtgagga ttctatactg atttgaagtg gagctgttca gaactgaacc 2400
ttgtaggaaa ttccaagggc ctttctactg aatctggtga tggggtgggg ccgtggcact 2460

ttctctgcca cagctgttct tcacagtgtt ggtgctaatg aggccagggt gcagggttcg 2520
attcacacgt aggccagtta acttagagaa aatctatttc cttacctcta gccagtcact 2580
tcctttttcc gcagttgtga tgggttttgc tgagccatcc actctgactg atttcctctg 2640
aagtaaacat atttacaatc caaagcaatt ctactgacag aagtgttgcc ttcataatca 2700
aacagcttgt ttttccatct cctctgcaac cctaattaaa tgagtacagg tctacaaaat 2760
gttttcaagg agaaaagcag catatcctta agtgaagtat tatatttttc aataaccctg 2820
tagtggcttg atgcagggaa ccctggggga ctttcagcga agagctgtgc tcttttctga 2880
ctagattaga gcgtttggag tggaagacgt caaatgtgta gtgagatgga ggttttacat 2940
tgttcttcta ctggctgtga tgaagtgcca gaatgtctct ttagaacaag agttagattc 3000
cccctttctc cttattgccc cttccgtttt gacttcccct ttatttattt gttgtctaat 3060
taggggccaa gtctgtaaag ttttgtcaaa gtgagttaga agttgttttc tcttactatt 3120
tgtgtttacc agagttggga gataagatag tttccatgaa ggtgtgtatg ttttatacga 3180
tgtttgttat agggccatgc attggtaact tgaaaataga ccagcttaat gtcttcagga 3240
tgtaaaactc tgaatacacg gcgtctcttt ttcatacatt gcatgtaagt tgttagtacc 3300
tcacaagcta cagaagttca gccatgagat tttgtttggc aacatgaaca gatttgtgta 3360
taactgcaat ggcctttttt tccagatttc cttattgact ttttgtttgc cttacctggg 3420
gctagttttt tatgctttgt acctagaaaa caaaaaatta cattcgttgg gctttttttc 3480
aaggttggga ttaccacacc acctggaata tcatactgtg gtttctgcct aaaattggca 3540
catgtaagta ttgaagaaaa tggttatata attcagttga aactcttggt tattagatgt 3600
taggcatctc ctgtatgtaa gacacaaggc caaccacaac acagaacgat gttgacctgt 3660
taagtattct ctgaaacatg gccaaaatgc attttatgag cttttttttt tgctattgta 3720
aatattagtg gtttacaatg cgctttagac atatttcttt aaaatgcaag cagtgagaaa 3780
taagacctct ctgaattagt agctctaaac tgttaacata gaatgttact tggaaaaagt 3840
ctggaatatg tggtgtacac aagcagtgct tcgtgaatga gtttcttagc ttttatagtg 3900
cgccatgttt ctcaaagttt gtttttgttg acaaaacatt ttataatata tatcttatgt 3960
ttattttttt tctcaactaa ttgtgtactg cactgtaagg tgaaaattag ccatccatta 4020
tttatcttct gtggcaatgc atttatatgg ttgattgggt ggggaatttt ttgcagaaag 4080
atgcaaagtg attgggtttt cgacttccta tcgcagggag cttttaagaa atattaattt 4140
cctatacatt tttccaatcc ccatgcaaac tgttcctgtt tacatacctt ctctgttgta 4200
tcagtacttt gagtgagaag acagtttatt taaaacttga gcaggctgtt cagcattgtt 4260
tctgcttctg aaatctgtat agtacactgg tttgtaatca ttatgtcttc attgaaatcc 4320
ttgctacttc tcttcctcct caatgaaata cattatatat tatctttatg tactcttaag 4380
aaaaacgagc aaggaagagt atcttcatta ttctcatttt ctctgagttg gaaacaaaaa 4440
catgaaggac tccaactaga agacagatat ttacatttaa atagattagt gggaaaactt 4500
taagagtttc cacatattag ttttcatttt ttgagtcaag agactgctcc ttgtactggg 4560

agacactagt agtatatgtt tgtaatgtta ctttaaaatt atctttttat tttataaggc 4620

ccataaatac tggttaaact ctgttaaaag tgggccttct atcttggatg gtttcactgc 4680

catcagccat gctgatatat tagaaatggc atccctatct acttacttta atgcttaaaa 4740

ttatacataa aatgctttat ttagaaaacc tacatgatac agtggtgtca gccttgccat 4800

gtatcagttt cacttgaaat ttgagaccaa ttaaatttca actgtttagg gtggagaaag 4860

aggtactgga aaacatgcag atgaggatat cttttatgtg caacagtatc ctttgcatgg 4920

gaggagagtt actcttgaaa ggcaggcagc ttaagtggac aatgttttgt atatagttga 4980

gaattttacg acacttttaa aaattgtgta attgttaaat gtccagtttt gctctgtttt 5040

gcctgaagtt ttagtatttg ttttctaggt ggacctctga aaaccaaacc agtacctggg 5100

gaggttagat gtgtgtttca ggcttggagt gtatgagtgg ttttgcttgt attttcctcc 5160

agagattttg aactttaata attgcgtgtg tttttttttt ttttaagtgg ctttgttttt 5220

ttttctcaag taaaattgtg aacatatttc ctttataggg gcagggcatg agttagggag 5280

actgaagagt attgtagact gtacatgtgc cttcttaatg tgtttctcga cacatttttt 5340

ttcagtaact tgaaaattca aaagggacat ttggttaggt tactgtacat caatctatgc 5400

ataaatggca gcttgttttc ttgagccacg gtctaaattt tgtttttata gaaatttttt 5460

atactgattg gttcatagat ggtcagtttt gtacacagac tgaacaatac agcactttgc 5520

caaaaatgag tgtagcattg tttaaacatt gtgtgttaac acctgttctt tgtaattggg 5580

ttgtggtgca ttttgcacta cctggagtta cagttttcaa tctgtcagta aataaagtgt 5640

cctttaactt caaaaaaaaa aaaaaa 5666

<210> 5
<211> 20
<212> DNA
<213> Unknown

<220>
<223> artificial sequence

<400> 5
ctggagaggc gctggaacat 20

<210> 6
<211> 19
<212> DNA
<213> Unknown

<220>
<223> artificial sequence

<400> 6
cgtgtaggta cggcagatc 19

<210> 7
<211> 21
<212> DNA
<213> Unknown

<220>
<223>  artificial sequence

<400>  7
ttcctccaac caagaaccag a                                                21


<210>  8
<211>  23
<212>  DNA
<213>  Unknown

<220>
<223>  artificial sequence

<400>  8
agtaggtgac tcttcactga tgt                                              23


<210>  9
<211>  20
<212>  DNA
<213>  Unknown

<220>
<223>  artificial sequence

<400>  9
ctaccacatc caaggaaggc                                                  20


<210>  10
<211>  20
<212>  DNA
<213>  Unknown

<220>
<223>  artificial sequence

<400>  10
ttttcgtcac tacctccccg                                                  20


<210>  11
<211>  21
<212>  DNA
<213>  Unknown

<220>
<223>  artificial sequence

<400>  11
guagucccag aguggaagct t                                                21

## Description

### Technical field

The present invention refers to a compound for use in treatment of prostate cancer, a method for in vitro diagnosing a prostate cancer and a method of screening for compounds modulating the TP53INP1 activity.

Therefore, the present invention has utility in the medical and pharmaceutical fields.

In the description below, the references into brackets (**[ ]**) refer to the listing of references situated at the end of the text.

### Background of the Invention

Prostate cancer is the most common malignancy in males and the second leading cause of cancer-related deaths in the United States and Europe (Gronberg H. Prostate cancer epidemiology. Lancet 2003;361:859-864, **[1]**). It appears in three steps: i) an initial prostate carcinogenesis step during which inflammatory phenomenoms seem involved, ii) an androgen-dependent progression step during which prostate cancer is not very agressive and responds to therapy iii) and a final androgen-independent step during which prostate cancer escapes to hormonal therapy (HNT) and leads very rapidly to death. The multi-step model of prostate carcinogenesis suggests an accumulation of genetic alterations, epigenetic changes and posttranslational modifications. Some authors focusing on the initial step claim that proliferative inflammatory atrophy is the precancerous lesion leading to Prostatic Intraepithelial Neoplasia (PIN) lesions and then to invasive carcinoma (De Marzo AM, Marchi VL, Epstein JI, Nelson WG, "Proliferative inflammatory atrophy of the prostate: implications for prostatic carcinogenesis", Am J Pathol 1999;155:1985-1992, **[2]** ; Nelson WG, De Marzo AM, Isaacs WB, "Prostate cancer", N Engl J Med 2003;349:366-381, **[3]** ; Putzi MJ, De Marzo AM, "Morphologic transitions between proliferative inflammatory atrophy and high-grade prostatic intraepithelial neoplasia" Urology 2000;56:828-832, **[4]**). In addition, TP53 mutations are observed in advanced PC (Stapleton AM, Timme TL, Gousse AE, et al., "Primary human prostate cancer cells harboring p53 mutations are clonally expanded in metastases", Clin Cancer Res 1997;3:1389-1397, **[5]**).

There are different types of treatment for patients with prostate cancer. Some treatments are standard and are currently used, and some are being tested in clinical trials.

Four types of standard treatment are used. The first one is watchful waiting, that is closely monitoring a patient's condition without giving any treatment until symptoms appear or change. This is usually used in older men with other medical problems and early-stage disease.

The second one is surgery, usually offered to patients in good health as treatment for prostate cancer.

The third one is radiation therapy, that uses high-energy x-rays or other types of radiation to kill cancer cells or keep them from growing. There are two types of radiation therapy. External radiation therapy uses a machine outside the body to send radiation toward the cancer. Internal radiation therapy uses a radioactive substance sealed in needles, seeds, wires, or catheters that are placed directly into or near the cancer. The way the radiation therapy is given depends on the type and stage of the cancer being treated. However, there is an increased risk of bladder cancer and/or rectal cancer in men treated with radiation therapy., and impotence and urinary problems may occur in men treated with radiation therapy.

The fourth one is hormone therapy, that removes hormones or blocks their action and stops cancer cells from growing. Hormone therapy used in the treatment of prostate cancer may include the following: (i) Luteinizing hormone-releasing hormone agonists that can prevent the testicles from producing testosterone, (ii) antiandrogens that can block the action of androgens, (iii) drugs hormones that can prevent the adrenal glands from making androgens, including ketoconazole and aminoglutethimide, (iv) orchiectomy that is a surgical procedure to remove one or both testicles, to decrease male hormone production, and (v) estrogens that can prevent the testicles from producing testosterone. However, estrogens are seldom used today in the treatment of prostate cancer because of the risk of serious side effects.

However, hot flashes, impaired sexual function, loss of desire for sex, and weakened bones may occur in men treated with hormone therapy. Other side effects include diarrhea, nausea, and pruritus (itching).

This is the reason why new types of treatment are being tested in clinical trials, as cryosurgery, to freeze and destroy prostate cancer cells. This type of treatment is also called cryotherapy. However, impotence and leakage of urine from the bladder or stool from the rectum may occur in men treated with cryosurgery.

Chemotherapy is used to try to stop the growth of cancer cells, either by killing the cells or by stopping them from dividing.

Biologic therapy is a treatment that uses the patient's immune system to fight cancer. Substances made by the body or made in a laboratory are used to boost, direct, or restore the body's natural defenses against cancer.

High-intensity focused ultrasound is a treatment that uses ultrasound (high-energy sound waves) to destroy cancer cells. To treat prostate cancer, an endorectal probe is used to make the sound waves.

Proton beam radiation therapy is a type of high-energy, external radiation therapy that targets tumors with streams of protons.

However, these new treatments are still at the stage of the trial, and their efficacy has not yet been proven.

Accordingly, a need exists to develop new tools for the treatment of prostate cancer.

### Description of the Invention

Tumour protein 53-induced nuclear protein 1 (TP53INP1) is a tumor suppressor gene, located on the chromosome band 8q22 (Nowak J, Tomasini R, Mattei MG, et al. "Assignment of tumor protein p53 induced nuclear protein 1 (TP53INP1) gene to human chromosome band 8q22 by in situ hybridization". Cytogenet Genome Res 2002;97:140E, **[6]**). Its expression depends on the activation of wilde-type p53 (Tomasini R, Samir AA, Carrier A, et al. "TP53INP1s and homeodomain-interacting protein kinase-2 (HIPK2) are partners in regulating p53 activity". J Biol Chem 2003;278:37722-37729 **[7]**), and so is a p53-inducible cell stress response protein.

In pancreas it has been shown that a micro-RNA (mi-RNA155) (Gironella M, Seux M, Xie MJ, et al. "Tumor protein 53-induced nuclear protein 1 expression is repressed by miR-155, and its restoration inhibits pancreatic tumor development". Proc Natl Acad Sci U S A 2007;104:16170-16175, **[8]**) could repress the expression of TP53INP1, and previous works showed that TP53iNP1 expression is strongly induced *in vivo* in mice with acute pancreatitis (Tomasini R, Samir AA, Vaccaro MI, et al. "Molecular and functional characterization of the stress-induced protein (SIP) gene and its two transcripts generated by alternative splicing. SIP induced by stress and promotes cell death". J Biol Chem 2001;276:44185-44192 **[9]**), and *in vitro* in several cell lines submitted to various stress agents (Okamura S, Arakawa H, Tanaka T, et al. p53DINP1, a p53-inducible gene, regulates p53-dependent apoptosis. Mol Cell 2001;8:85-94, **[10]** ; Tomasini R, Samir AA, Pebusque MJ, et al. « P53-dependent expression of the stress-induced protein (SIP). Molecular and functional characterization of the stress-induced protein (SIP) gene and its two transcripts generated by alternative splicing. SIP induced by stress and promotes cell death". EurJ Cell Biol 2002;81:294-301, **[11]**).

Whereas there is an overexpression of TP53INP1 in numerous tissues submitted to stress agents, TP53INP1 is downexpressed in stomach, pancreatic and inflammation-mediated colic carcinomas. In medullary thyroid carcinomas, TP53INP1 overexpression correlates with poor prognosis. Overexpression of TP53INP1 induces cell cycle arrest in G1 phase and enhances the p53-mediated apoptosis (**[7]**). Furthermore TP53INP1 is a major mediator of p53 antioxidant function (Cano CE, Gommeaux J, Pietri S, et al. "Tumor protein 53-induced nuclear protein 1 is a major mediator of p53 antioxidant function". Cancer Res 2009;69:219-226, **[12]**). Jiang, et al. (Jiang PH, Motoo Y, Garcia S, lovanna JL, Pebusque MJ, Sawabu N. "Down-expression of tumor protein p53-induced nuclear protein 1 in human gastric cancer". World J Gastroenterol 2006;12:691-696, **[13]**) showed a downexpression of TP53INP1 in gastric adenocarcinomas and its association with a poor prognosis. Gironella, et al. (**[8]**) also showed that TP53INP1 expression is decreased in pancreatic ductal adenocarcinomas. Gommeaux, et al. (Gommeaux J, Cano C, Garcia S, et al. "Colitis and colitis-associated cancer are exacerbated in mice deficient for tumor protein 53-induced nuclear protein 1". Mol Cell Biol 2007;27:2215-2228, **[14]**) showed that downexpression of TP53INP1 in a mice model was involved in inflammation-mediated colic carcinogenesis.

Before the works of the Applicant, investigation of the expression of TP53INP1 in prostate tissues has never been made, and TP53INP1 expression has never been reported in prostate cancer.

Surprisingly, the Applicant has found, after important investigations of variations of TP53INP1 expression and their correlation to clinicopathological parameters in prostate cancer, that TP53INP1 overexpression in prostate cancer is a worse prognostic factor, particularly predictive of biological cancer relapse, and that it is a relevant target for potential specific therapy.

Interestingly, the Applicant showed for the first time there was a neo-expression of TP53INP1 in prostate luminal cells in inflammatory tissues, in high grade Prostatic Intraepithelial Neoplasia (PIN) lesions and in prostate cancer.

Furthermore, in prostate cancer the Applicant showed that TP53INP1 overexpression correlated with poor prognostic factors such as high Gleason grade and score, unfavourable d'Amico score, lymph node invasion and biological cancer relapse.

Moreover, the Applicant demonstrated for the first time that TP53INP1 overexpression was an independent predictive factor of biological cancer relapse on multivariate analysis. That is very interesting for early detection of patients with a poor outcome in order to treat them more intensively.

The findings of the Applicant in human prostate cancer contrast with the pattern usually observed in others neoplasms where TP53INP1 expression inversely correlates with tumour aggressivity (Jiang, et al. **[13]**, Gommeaux, et al. **[14]**).

Accordingly, in a first aspect, the invention provides for a compound inhibiting TP53INP1 activity, for use in treatment of prostate cancer.

"TP53INP1" refers herein to the gene, the RNA transcript or the protein TP53INP1.

"TP53INP1 activity" refers herein to any activity or function of TP53INP1 gene, RNA transcript or protein in prostate cancerous or non cancerous cells. This activity may be the induction of p53-inducible cell stress response normally induced by TP53INP1 in prostate cancer cells, TP53INP1 expression, TP53INP1 gene transcription or translation, TP53INP1 activation of autophagy and a putative action of this molecule on the regulation of androgen receptor activity during prostate cancer development.

"Compound inhibiting TP53INP1 activity "or "Inhibitor of TP53INP1 activity "refers herein to any chemical that prevents TP53INP1 gene, transcript or protein to realise its function in prostate cancer cells. The inhibition may be the inhibition of the transcription of the gene, the inhibition of the translation of mRNA, or the inhibition of the expression or function of the protein.

For example, the compound inhibiting TP53INP1 activity may be chosen among the compounds comprising: anti-sense oligonucleotides, more particularly siRNA ("small interfering RNA"), miRNA (microRNA), RNAi (RNA interference) or shRNA oligonucleotides (short hairpin RNA), antibodies, peptides and chemical inhibitors.

According to the present invention, the inhibition may be a total or partial inhibition of TP53INP1 activity.

"Partial inhibition" refers herein to an inhibition or a decrease of at least 10% of TP53INP1 activity in a cancer cell compared to the normal level of TP53 activity in the same prostate cancer cell. The partial inhibition may be an inhibition of at least 20%, 30%, 40%, 50%, 60%, 70%, 80% or 90% of TP53INP1 activity in a cancer cell compared to the normal level of TP53 activity in the same prostate cancer cell.

"Total inhibition" refers herein to an inhibition or decrease of almost 100% of TP53INP1 activity in a cancer cell compared to the normal level of TP53INP1 activity in the same prostate cancer cell.

Any method known by the man skilled in the art may be used in order to measure the inhibition, for example quantitative PCR or Western blot, as described by Rocchi et al. (**[29]**) and by Tomasini et al. (Tomasini et al Oncogene. 2005 Dec 8;24(55):8093-104. **[30]**).

The inhibition may be a specific inhibition. "Specific inhibition" refers herein to an inhibition that only targets prostate cancer cells, without or with very few side effect on non-cancerous prostate cells.

The compound may bind to TP53INP1 protein or to TP53INP1 transcript. Advantageously, the binding allows the inhibition of TP53INP1 activity.

Preferentially, the compound may bind to TP53INP1β (TP53INP1 beta) RNA transcript or protein isoform. Indeed, TP53INP1 protein is present in normal/non cancerous cells as two isoforms : Alpha isoform of 18 kDa (SEQ ID NO: 2) and Beta isoform of 27 kDa (SEQ ID NO: 1). The RNA sequence of Beta isoform is SEQ ID NO: 3 (also corresponding to NCBI Reference Sequence: NM_033285.3). The RNA sequence of Alpha isoform is SEQ ID NO: 4. The Applicant has demonstrated for the first time that only the beta isoform is over-expressed in prostate cancer cells. Advantageously, the use of a compound binding to TP53INP1 beta transcript or protein isoform may allow to target only the prostate cancer cells, and has little or no effect on prostate non cancerous cells.

Preferentially, the compound may bind specifically to TP53INP1β transcript or protein isoform. This means that the compound does not bind to the TP53INP1α isoform. Advantageously, the inhibition of TP53INP1 activity is, in this case, a specific inhibition of the activity of TP53INP1β transcript or protein isoform.

The compound may bind to the sequence SEQ ID NO: 11 on TP53INP1β transcript. This is a Sense strand siRNA: GUAGUCCCAGAGUGGAAGCtt.

The compound may comprise the sequence SEQ ID NO : 5, or consist of the sequence SEQ ID NO : 5. Advantageously, this compound is a siRNA that binds specifically to TP53INP1β transcript isoform.

Another object of the invention is an inhibitor of TP53INP1 activity comprising or constiting to the sequence SEQ ID NO : 5.

Another object of the invention is a method for in vitro diagnosing a prostate cancer, comprising the step of determining the level of TP53INP1 activity in a patient-derived biological sample comprising a prostate cell, wherein an increase of said activity compared to the normal control level indicates that said subject suffers from prostate cancer.

" Normal control level" refers herein to the level of activity of TP53INP1 in a non-cancerous prostate cell. The activity may be the induction of p53-inducible cell stress response normally induced by TP53INP1 in prostate cancer cells, TP53INP1 gene transcription or translation, TP53INP1 activation of autophagy and a putative action of this molecule on the regulation of androgen receptor activity during prostate cancer development. The non-cancerous cell may be PNT1A or PNT2C2 (Lang, S.H. Br J Cancer. 2000 Feb;82(4):990-7, [31]), or any other known non-cancerous prostate cell line. Usually, the normal control level is determined by methods known by the man skilled in the art, such as Western blot or, quantitative PCR ([29], [30]).

« Increase » refers herein to an increase in the level of activity of at least 20%, or 30%, 40%, 50%, 60%, 70%, 80% 90%, 100% or more compared to the normal control level. Alternatively, the increase may be the passage from "negative" to "positive" at the time of transformation of the cells. The increase may be determined by qualitative methods, as Western blot or PCR.

"Patient-derived biological sample" refers herein to any biological sample removed from the patient and containing some prostate cancer cells. This may be a sample of lymph nodes, sample of prostate, surrounding tissue, seminal vesicles or metastasis as for example lymph nodes and bone liver.

The determination of the level of activity may be realised by the measurement of β isoform of TP53INP1. In this case, the measurement may be made by the measurement of the level of TP53INP1β mRNA (messenger RNA) or protein. This determination may be made by any method known in the art, as quantitative PCR quantitative, western blot, ELISA test elisa or in situ hybridation.

Another object of the invention is a method of screening for compounds modulating the TP53INP1 activity comprising the steps of:
a. contacting a test compound with TP53INP1 protein or TP53INP1 transcript, particularly TP53INP1β, or prostate cancer cell,
b. detecting the binding activity between TP53INP1 protein or TP53INP1 transcript and the test compound or the modulation of TP53INP1 activity in said cell,
c. selecting a compound that modulates the TP53INP1 activity in said cell or that binds to TP53INP1 protein or TP53INP1 transcript.
"Modulating the TP53INP1 activity" refers herein to an increase or decrease of the TP53INP1 activity compared to the TP53INP1 activity of control cancerous or non-cancerous cells, or to the TP53INP1 activity of the cell prior to the contact with the test compound, or to the binding of a test compound to TP53INP1. For example, the control cell may by LNCaP (ATCC CRL-1740) or C4-2 (Wu HC, Hsieh JT, Gleave ME, Brown NM, Pathak S, Chung LW. Deri- vation of androgen independent human LNCaP prostatic cancer cell sublines : role of bone stromal cells. Int J Cancer 1994 ; 57 : 406-12, [32]). Advantageously, the TP53INP1 activity depends on the efficacy of the compound to test compound to modulate TP53INP1 activity compared with the level of activity in control cell. The increase or the decrease may be at least of 10%, or 20%, 30%, 40%, 50%, 60%, 70%, 80%, 90%, 100% or more.

"Test compound" refers herein to any compound or part of compound likely to modulate the TP53INP1 activity. The compound may be for example a nucleic acid, an amino acid, a peptide, a protein, a chemical compound, a carbohydrate, a lipid.

The step of contacting may be realised in conditions adapted to allow the binding between the test compound and the TP53INP1 protein or transcript, or to allow the modulation to occurs. These conditions may be easily determined by the man skilled in the art according to his general knowledge of the art.

Another object of the invention is a pharmaceutical composition comprising a compound as defined above, and a pharmaceutically acceptable vehicle.

Another object of the invention is a method of treatment of a patient in need thereof, comprising the step of administering to the patient a pharmaceutically effective dose of a compound or a pharmaceutical composition as defined above.

The administration can be made by using any pharmaceutical way known by the skilled person and useful to administrate a compound or pharmaceutical composition as defined above. Administration may be carried out directly, i.e. pure or substantially pure, or after mixing of the compound with an pharmaceutically acceptable carrier and/or medium. The pharmaceutical composition may be in a form selected from the group comprising an injectable form, syrup, oral suspension, a pellet, powder, granules, spray, transdermal patch or local form (cream, lotion, collyrium).

The pharmaceutically acceptable carrier may be any known pharmaceutically carrier used for the administration of the compound. For example, this carrier may be selected from the group comprising for example the monomethoxy-polyethyleneglycol (for exemple as in Viraferonpeg®) or Liposome (for exemple as in Ambizome®).

The administration may be defined so allow delivery of a pharmaceutically acceptable and efficient dose for the treatment of prostate cancer. For example, the administration may comprise a dose of 1 to 5 mg/kg of body weight per day, for example of 2 to 4 mg/kg of body weight per day.

The administration may be carried out with one dose or with a plurality of doses per day.

The invention is further illustrated by the following examples with regard to the annexed drawings not be construed as limiting.

### Description of the Figures

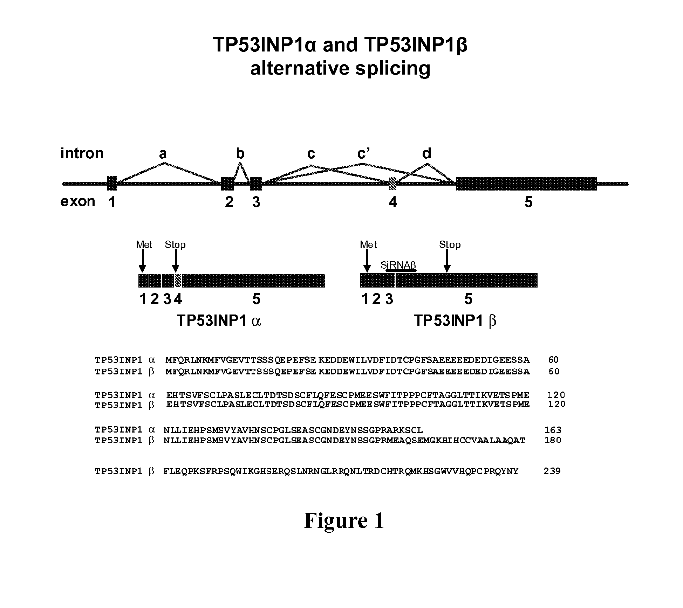

Figure 1 shows the alternative splicing of TP53INP1 gene, the TP53INP1α and TP53INP1β sequences, and the localisation of the binding of siRNAβ on TP53INP1β transcript.

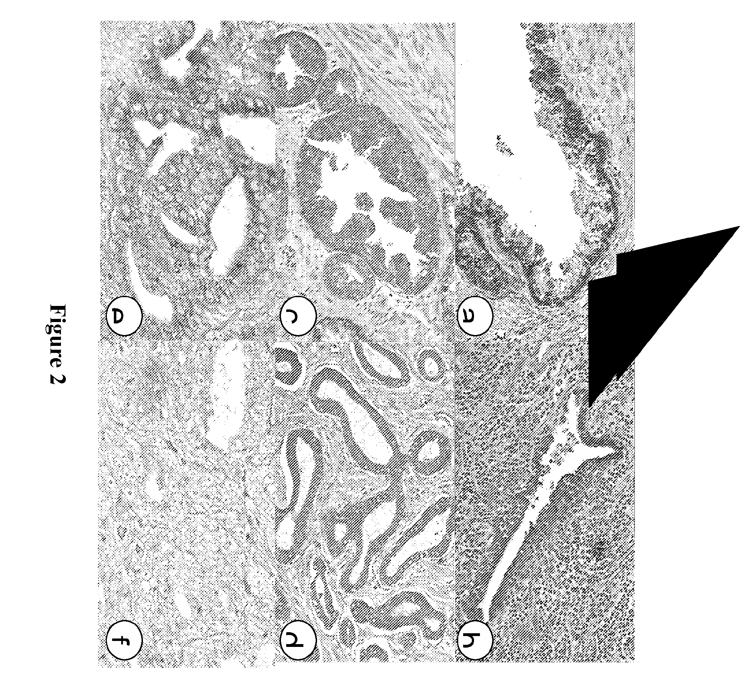

Figure 2 shows TP53INP1 expression (IHC, magnificationx200). In normal prostate tissues, TP53INP1 was only expressed in the cytoplasm of prostate basal cells (black arrows) (a). There was a neo-expression of TP53-INP1 in the cytoplasm of prostate luminal cells in inflammatory prostate tissues (b), in high grade PIN lesions (c) and in PC (d). CISH: expression of TP53INP1 digoxigenin-labelled antisense. TP53INP1 was localized in cytoplasm and nucleoli of the prostate tumour cells (e). CISH : TP53INP1 sense probe (negative control) (f).

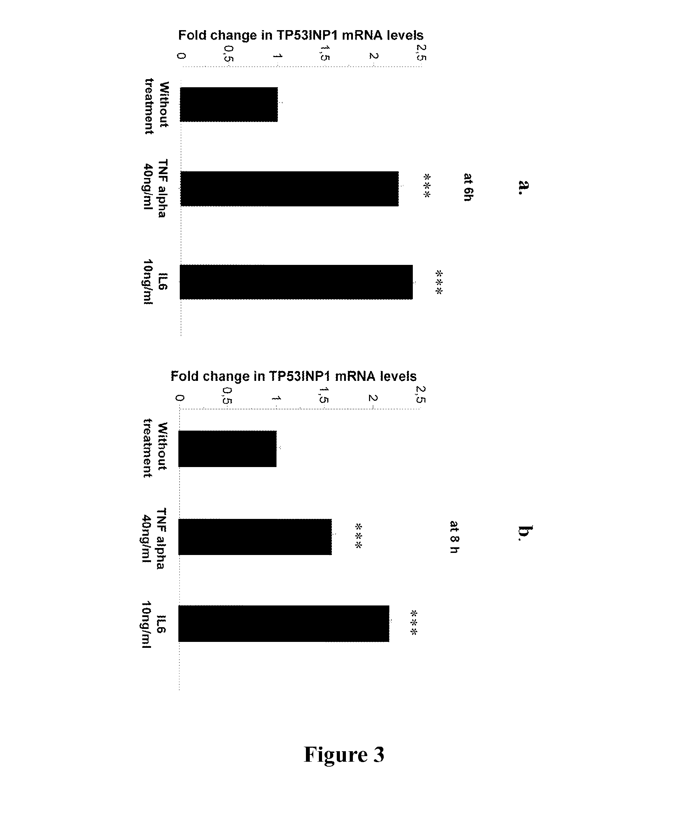

Figure 3 shows treatment of LNCaP cells *in vitro* with TNFalpha or IL6 increased the TP53INP1 mRNA levels. LNCaP cells were treated with 40ng/ml TNFalpha or 10ng/ml IL6. In each case (without treatment, TNFalpha and IL6), cells were harvested for quantitative Real-Time PCR (6h on figure 3a, and 8h on figure 3b). TP53INP1 mRNA levels were normalized to 18S levels, and the fold change between treated and untreated cells are shown. Relative to untreated cells, treatment of LNCaP cells with 40ng/ml TNFalpha or 10ng/ml IL6 increased the TP53INP1 mRNA levels by 2.25- or 2.41- fold respectively (***p≤0.001) after 6h and by 1.57- or 2.17- fold respectively (***p≤0.001) after 8h.

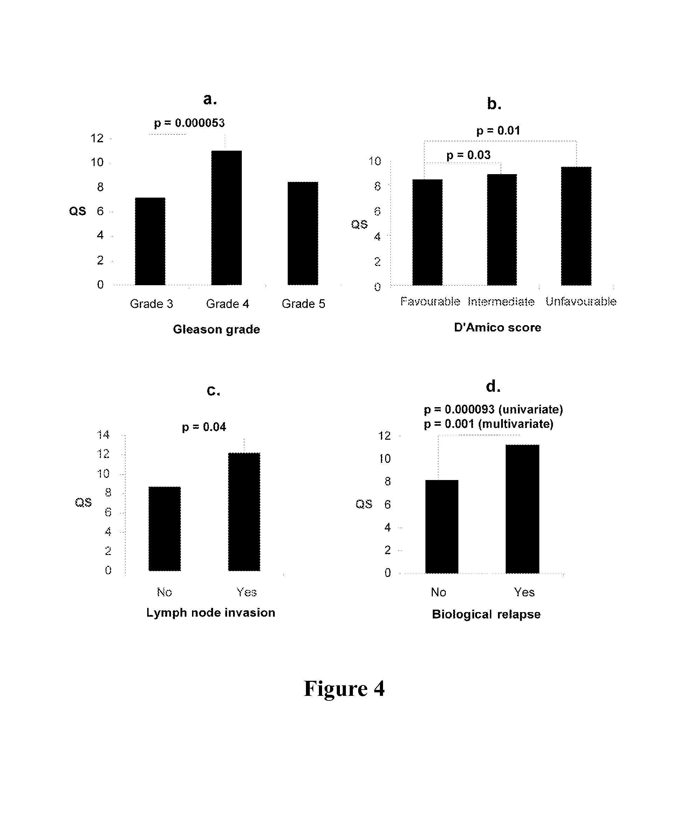

Figure 4 shows in human PC, TP53INP1 overexpression correlates with poor prognostic factors and is an independent predictive factor of biological cancer relapse. TP53INP1 expression was significantly higher in Gleason grade 4 tumours compared with Gleason grade 3 tumours (p=0.0000053)(a). On univariate analysis, increase of TP53INP1 expression was significantly associated with unfavourable D'Amico score (p=0.01) (b), lymph node invasion (p=0.04) (c) and biological cancer relapse (p=0.000093) (d). On multivariate analysis, TP53INP1 overexpression was an independent predictive factor of biological cancer relapse (p=0.001) (d).

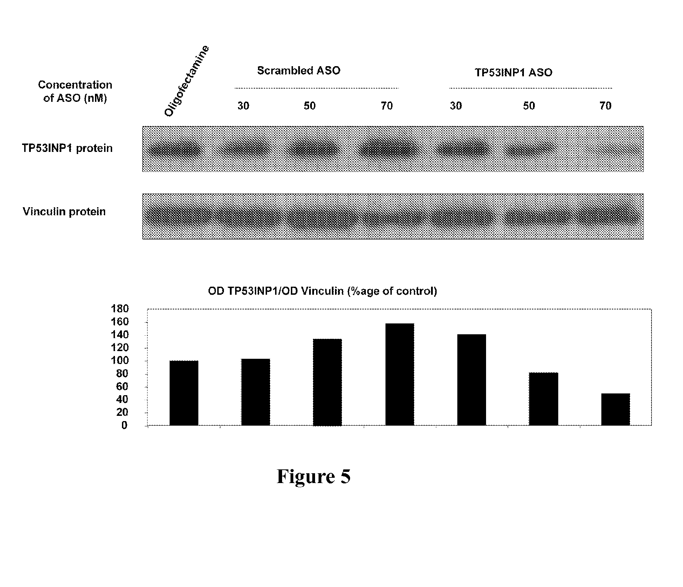

Figure 5 shows TP53INP1 is dose-dependently downregulated by oligonucleotide antisense (ASO) *in vitro*. LNCaP cells were treated with 10nM, 30nM, 50nM, 70nM and 100nM of ASO in order to obtain the minimal efficient dose (MED). Protein was extracted from transfected cells and TP53INP1 protein levels were analysed by western blotting using 40µg of protein. TP53INP1 ASO significantly reduced TP53INP1 protein expression up to 70% in a dose-dependent manner whereas TP53INP1 protein expression was not significantly suppressed by scrambled ASO control.

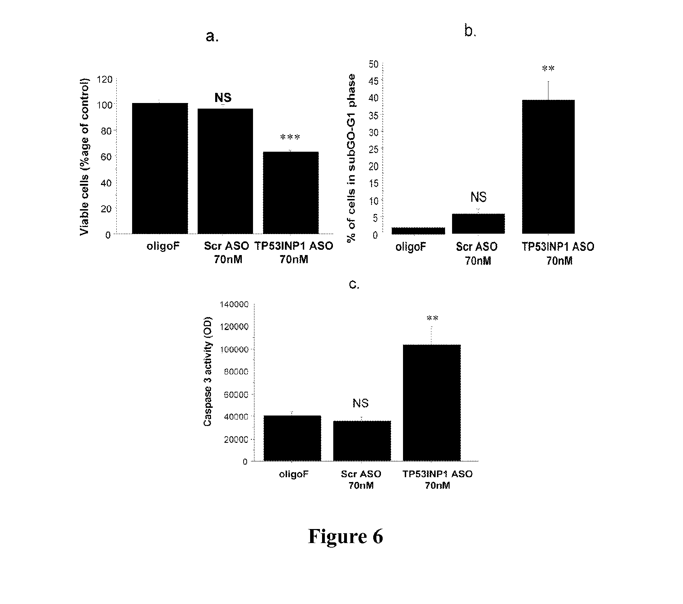

Figure 6 shows TP53INP1 ASO inhibits proliferation of LNCaP cells and induces apoptosis *in vitro*. LNCaP cells were treated daily with 70nM of TP53INP1 ASO or scrambled ASO control for 2 days. Cell viability was determined using the crystal violet assay. TP53INP1 ASO treatment significantly reduced LNCaP cell growth by 33.3%(***p≤0.001), 1 day post-treatment compared with scrambled ASO control (figure 6a). The induction of apoptosis by TP53INP1 ASO was clearly shown using flow cytometry. After the same treatment schedule described above, the fraction of cells undergoing apoptosis (sub-Go-G1 fraction) was significantly higher after treatment with TP53INP1 ASO compared with scrambled ASO control (39 % versus 5.77 %)(**p≤0.01) (figure 6b). The induction of apoptosis by TP53INP1 was also shown using the measurement of the caspase 3/7 activity. The caspase 3/7 activity was significantly higher after treatment with TP53INP1 ASO at a concentration of 70nM compared with scrambled ASO control (ratio = 2.94) (**p≤0.01) (figure 6c).

### Examples

The aim of the following studies was to analyse the expression patterns of TP53INP1 in a large series of human prostate cancer (PC) to (1) identify the variations of TP53INP1 expression; (2) investigate their correlation with clinicopathological parameters and evaluate their prognostic value; (3) assess the biological function of TP53INP1 downregulation using a TP53INP1 Antisense Oligonucleotide (ASO).

### Materials and methods

### Patients and specimens

PC samples for Tissue Micro-Array (TMA) were obtained from 91 patients who underwent Radical Prostatectomy (RP) in the Department of Urology of the Hôpital Nord of Marseille from January 2002 to December 2006. 51 patients had a favourable D'Amico score (D'Amico AV, Whittington R, Malkowicz SB, et al. "Predicting prostate specific antigen outcome preoperatively in the prostate specific antigen era". J Urol 2001;166:2185-2188 **[15]**), 9 had an intermediate score and 31 had an unfavourable score. Gleason score varied from 6 to 9: 20 patients were at 6, 56 patients were at 7, 10 patients were at 8 and 5 patients were at 9. 48 patients were pT2 (palpable tumor localised at prostate) and 43 patients were pT3 (tumor with extensions outside the prostatic capsule). 4 patients had lymph node metastasis and 18 patients had a biological cancer relapse with a mean follow-up of 71 months (37 months-95 months). Clinicopathological data are summarized in Table 1.

**Table 1 : Clinicopathological parameters of the TMA's patients**

| Clinicopathological parameters | Number of patients (N=91) |
|---|---|
| D'Amico score | |
| favourable | 51 |
| intermediate | 9 |
| unfavourable | 31 |
| | |
| Gleason score | |
| 6 | 20 |
| 7 | 56 |
| 8 | 10 |
| 9 | 5 |
| | |
| Tumour stage (pT) | |
| pT2 | 48 |
| pT3 | 43 |
| | |
| Lymph node invasion | |
| No | 87 |
| Yes | 4 |
| | |
| Biological relapse | |
| No | 73 |
| Yes | 18 |

All RP were entirely included according to the Stanford technique (Stamey TA, McNeal JE, Freiha FS, Redwine E. Morphometric and clinical studies on 68 consecutive radical prostatectomies. J Urol 1988;139:1235-1241, **[16)**]. All tumour samples were fixed in buffered formalin, paraffin-embedded at controlled temperature and stored at 18-22°C. All patients were informed at the time of surgery that tissues blocks stored in the laboratory archives might be used for delayed diagnostic and research purpose, to which they agreed.

### TMA construction

The TMA was constructed according to the following protocol. For each Gleason grade, two cores were realised from the primary paraffin block (donor block). Tumours areas were delimited by circling within tissue section appropriate areas with a permanent black pen on hematoxylin and eosin-stained (HES) paraffin sections in order to guide the technician's punches of cores from the primary paraffin block. Cores were sampled using a tissue arreyer (Alphelys, Plaisir, France). Core cylinders of 0.6mm diameter punched from the donor block were then deposited in the recipient paraffin block. 16 normal prostate tissues, 6 PIN lesions and 6 negative controls (placenta) were introduced. We punched two cores per Gleason grade (i.e. 2, 4 or 6 cores per patient) with a total of 330 cores. TMA sections (4µm thick) were cut 24 hours before immunohistochemical processing.

### Immunohistochemistry (IHC)

A standard avidin- biotin- peroxydase complex method (ABC) was used for immunostaining. Deparaffined sections were treated by microwaving at a high power for 5 min twice in a 10mmol/L citrate buffer to retrieve antigenecity. After washing with PBS, the sections were immersed in 3% hydrogen peroxide in methanol for 20 min to block any endogenous peroxides activity. Then the ABC staining system kit (Santa Cruz Biotechnology Inc., Santa Cruz, CA, USA) was used for the detection. Sections were incubated overnight at 4°C with 6 µg/mL of rat anti-human monoclonal antibody raised against TP53INP1 (clone E12 kindly provided by Dr A. Carrier **[8]**). After washing with PBS, detection was done by successively incubating the sections with biotinylated goat anti-rat IgG for 30 min, and avidin-biotin-HRP for 30 min. After extensive washings with PBS (Phosphate Buffer Saline), sections were stained with 3,3-diaminobenzidine for 2-10 min. Then, sections were counterstained with hematoxylin, dehydrated, and mounted.

### Chromogenic In Situ Hybridization (CISH)

Paraffin blocks and HES slides from 20 radical prostatectomy specimen were obtained from Vancouver General Hospital. Paraffin blocks were chosen by observing the HES slides. Small TMA (60 cores-triplicates) were constructed from these blocks. In an RNase free environment, paraffin blocks were cut into 5-micron sections and stored in 4C°. The paraffin sections were dewaxed in 2 Caplan Jars of Xylene (LABOVERT, Montpellier, France) each for 10 minutes and re-hydrated using a graded set of ethanol solutions. After sections were treated with Proteinase K (20ug/ml) at 37 C° for 30 minutes, they were acetylated by incubation in 0.1M tri-ethanolamine pH 8.0 buffer containing 0.25% acetic anhydride for 10 minutes. Sections were incubated in pre-hybridization buffer containing 50% formamide for 10 minutes at 37 C°. Riboprobes (sense and antisense) were obtained from Dr MJ Pebusque. Sense and antisense probes were labeled using DIG- RNA Labeling Kit (Sp6/T3) from Roche (Roche Applied Science, Penzberg, Germany). Sections were hybridized with probes (8.3ng/100ul in hybridization buffer) overnight at 42C°. The next day sections were washed by 2 x SSC (saline-sodium citrate), 1x SSC and 0.1 x SSC solutions consecutively for 2 x 15 minutes in each. The sections incubated with NTE (NaCl, Tris-HCl, EDTA) buffer containing RNase A for 30 minutes to remove unattached RNA. After wash in 0.1 X SSC, sections were incubated in posthybridization buffer containing 0.1% triton and 2% BSA for 30 minutes. Anti-Dig alkaline phosphotase-Fab fragment from Roche (Roche Applied Science, Penzberg, Germany) in dilution of 1/100 was applied on sections for 2 hours. After 3 washes in post-hybridization buffer 1 and incubation in post-hybridization buffer 2 for 10 minutes sections were transferred to colour solution of NBT/BCIP (nitro blue tetrazoluim salt plus 5- bromo-4 chloro indolyl phosphate) from Roche (Roche Applied Science, Penzberg, Germany) and were incubated overnight in room temperature in dark. The next day sections were washed with post-hybridization buffer 3 for 5 minutes, rinsed in DW and mounted cover glass with aqueous mounting media (Faramount, DakoCytomatio, Carpenteria, Ca, USA).

### Image analysis procedure

Image analysis was used for quantification of immunohistochemistry. The TMA analysis with the SAMBA 2050 automated device (SAMBA Technologie / TRIBVN, Châtillon 92320, France) was performed as previously described (Charpin C, Garcia S, Bonnier P, et al. Prognostic significance of Nm23/NDPK expression in breast carcinoma, assessed on 10-year follow-up by automated and quantitative immunocytochemical assays. J Pathol 1998;184:401-407 **[17]** ; Charpin C, Garcia S, Bonnier P, et al. bcl-2 automated and quantitative immunocytochemical assays in breast carcinomas: correlation with 10-year follow-up. J Clin Oncol 1998;16:2025-2031 **[18]** ; Charpin C, Garcia S, Bouvier C, et al. Automated and quantitative immunocytochemical assays of CD44v6 in breast carcinomas. Hum Pathol 1997;28:289-296 **[19]** ; Charpin C, Garcia S, Bouvier C, et al. CD31/PECAM automated and quantitative immunocytochemical assays in breast carcinomas: correlation with patient follow-up. Am J Clin Pathol 1997;107:534-541, **[20]** ; Dales JP, Garcia S, Carpentier S, et al. Long-term prognostic significance of neoangiogenesis in breast carcinomas: comparison of Tie-2/Tek, CD105, and CD31 immunocytochemical expression. Hum Pathol 2004;35:176-183, **[21]** ; Dales JP, Garcia S, Meunier-Carpentier S, et al. Overexpression of hypoxia-inducible factor HIF-1alpha predicts early relapse in breast cancer: retrospective study in a series of 745 patients. Int J Cancer 2005;116:734-739, **[22]** ; Charpin C, Vielh P, Duffaud F, et al. Quantitative immunocytochemical assays of P-glycoprotein in breast carcinomas: correlation to messenger RNA expression and to immunohistochemical prognostic indicators. J Natl Cancer Inst 1994;86:1539-1545, **[23]** ; Altman DG, Lausen B, Sauerbrei W, Schumacher M. Dangers of using "optimal" cutpoints in the evaluation of prognostic factors. Je Natl Cancer Inst 1994;86:829-835, **[24]** ; Charpin C, Giusiano S, Secq V, et al. Quantitative immunocytochemical profile to predict early outcome of disease in triple-negative breast carcinomas. Int J Oncol 2009;34:983-993, **[25]** ; Charpin C, Secq V, Giusiano S, et al. A signature predictive of disease outcome in breast carcinomas, identified by quantitative immunocytochemical assays. Int J Cancer 2009;124:2124-2134, **[26]** ; Garcia S, Dales JP, Charafe-Jauffret E, et al. Poor prognosis in breast carcinomas correlates with increased expression of targetable CD146 and c-Met and with proteomic basal-like phenotype. Hum Pathol 2007;38:830-841, **[27]**)**.**

First, an image of the entire slide was built up using a low-power magnification (X2, pixel dimension 3.7µm). Second, the area of the slide containing the TMA cores was automatically delineated and scanned at higher magnification (X10, pixel dimension 7.4 µm). Third, a specially developed tool referred to as TMA crop™ then allowed superimposition of the TMA grid onto the reduced image and precise alignment of each node of the grid with the core location within the image. The final step was performed automatically using the core image contents to ensure pixel precision of the match. From the images acquired with X10 magnification, a new set of images was next computed, one for each core. After colour analysis of the core images, the SAMBA 'immuno' software was applied (**[24-27]**) in usual tissue sections. Several parameters per core were computed : the area of counterstaining, the ratio of the positive area versus counterstained areas and a quick score QS (percentage of positive area X mean optical density). Optical density (OD) was evaluated on a scale of grey levels (arbitrary units ranging from 0 (100% transmission, OD= 0) to 255 (1 % transmission, OD=2)). The computation of each parameter was obtained provided numerical values consisting of continuous variables for statistical tests.

### Tumour cell lines

Human prostate cancer LNCaP cells were purchased from the American Type Culture Collection (Manassas, VA). Cells were maintained in RPMI 1640 (Life Technologies, Inc., Gaithersburg, MD) supplemented with 10% FBS.

### Treatment of LNCaP cells with cytokines

Cells were plated at a density of 1million per 78cm2 in 10ml RPMI (Roswell Park Memorial Institute medium) growth medium (Life Technologies, Inc., Gaithersburg, MD). After 24 h the cells either remained untreated or were treated (stimulated) with either 40ng/ml TNFalpha or either 10ng/ml IL6. In each case (without treatment, TNFalpha and IL6), reaction was stopped and cells were frozen at -80 °C at 6h and 8h for RNA extraction.

### RNA extraction

Total RNA was extracted directly from cells using TRIZOL reagent (Invitrogen).

### Quantitative Real-Time PCR Analysis

The expression of the TP53INP1 mRNAs was analysed by quantitative Real-Time PCR amplification analysis. Total RNAs were isolated using Trizol method (Invitrogen). Then 1 µg RNAs were reverse-transcribed into cDNA by using ImProm-II™ Reverse Transcription system (Promega, Madison, Wisconsin, USA) (0.5 µg oligo dT primer, 1 µL ImProm-II™ reverse transcriptase, 0.5 µL of RNase inhibitor, 1 µL of 10 mM dNTP mix, 4.8 µL of 25 mM MgCl2, 4 µL of 5xRT buffer) in a final volume of 20 µL. The thermal cycling protocol employed included two steps (step 1: 70 °C for 5 min, and 4 °C for 5 min; step 2: 25 °C for 5 min, 42 °C for 1 h, 70 °C for 15 min, and 4 °C forever).

The quantitative Real-Time PCR was conducted using the LightCycler 2.0 instrument (Roche Diagnostics, Meylan, France). Reaction mixtures contained total volume of 20 µl consisting of: 5 µl of each diluted cDNA (1:10), 10 µL SYBR Premix Ex Taq (2X) (TaKaRa Bio. Inc, Japan), 0.4 µL primers Forward and primers Reverse (10 µM) of target gene TP53INP1 and internal control 18S, and 4.2 µL H2O. The sequences of primers are as following: TP53INP1 primer F: 5'-TTCCTCCAACCAAGAACCAGA-3' (SEQ ID NO: 7) and primer R: 5'-AGTAGGTGACTCTTCACTGATGT-3' ((SEQ ID NO: 8); 18S primer F: 5'-CTACCACATCCAAGGAAGGC-3' (SEQ ID NO: 9) and primer R: 5'-TTTTCGTCACTACCTCCCCG-3 (SEQ ID NO: 10) (Eurogentec S. A., Seraing, Belgium). The PCR conditions were: an initial denaturation step at 95°C for 10 s; then 45 cycles of 95 °C for 5 s, 57 °C for 6 s, and 72 °C for 12 s. A melting curve was carried out after amplification programme by heating at temperatures from 65 °C to 95 °C in 1.5 min. And a final cooling step at 40 °C for 1 min was performed. Each sample was analysed in triplicate in the PCR reaction to estimate the reproducibility of data. The data were acquired by using Roche Molecular Biochemicals light cycler software version 3.5 and statistical analysis was obtained by RealQuant (Roche).

### TP53INP1 ASO (anti-sense oligonucleotides)

Phosphorothioate ASOs used in this study were purchased from Operon Biotechnologies (Cologne, Germany). The sequence of TP53INP1 ASO used corresponded to the human TP53INP1 translation initiation site (5'-CTGGAGAGGCGCTGGAACAT-3') (SEQ ID NO: 5), see figure 1. A scrambled antisense oligonucleotide ASO (5'-CGTGTAGGTACGGCAGATC-3') (SEQ ID NO: 6) was used as a control.

### Treatment of LNCaP cells with increasing concentrations of TP53INP1 ASO

LNCaP cells were plated at the density of 1 million cells per 78cm2 and treated 1 day later for 2 days with increasing concentrations of ASO in order to obtain the minimal efficient dose (MED). Oligofectamine, a cationic lipid (Invitrogen, Life Technologies) was used to increase the ASO uptake into the cells. LNCaP cells were treated with 10nM, 30nM, 50nM, 70nM and 100nM of ASO after a pre-incubation for 20 minutes with 3 mg/mL oligofectamine in serum free optiMEM. Fours hours after the beginning of the incubation, the medium was replaced with standard culture medium described above.

### Western Blot Analysis

Samples containing equal amounts of protein (40 µg) from lysates of treated LNCaP cells underwent electrophoreses on a SDS-polyacrylamide gel and transferred to a nitrocellulose filter. Filters were blocked in PBS containing 5% non fat milk powder at 4°C overnight and then incubated for 1 hour with a 1:200-diluted anti-human TP53INP1 rat monoclonal antibody (kindly provided by Dr A. Carrier **[8]**) or 1:2000-dilutated antihuman Vinculin mouse monoclonal antibody (Sigma Chemical Co., St Louis, MO). Filters were then incubated for 30 minutes with 1:1000-diluted horseradish peroxidase-conjugate antirat or 1:2500-dilutated horseradish peroxidase-conjugate antimouse monoclonal antibody (Santa Cruz Biotechnology, Inc., Santa Cruz, CA). Specific proteins were detected using an enhanced chemiluminescence western blotting analysis system (Amersham Life Science, Arlington Heights,IL).

### Treatment of LNCaP cells with 70nM of ASO (MED)

LNCaP cells were plated at the density of 100000 cells per 3.8 cm2 and 1 million per 78.5cm2 and treated 1 day later for 2 days with ASO. Oligofectamine, a cationic lipid (Invitrogen, Life Technologies) was used to increase the ASO uptake into the cells. LNCaP cells were treated with 70nM of ASO after a pre-incubation for 20 minutes with 3 mg/mL oligofectamine in serum free optiMEM. Fours hours after the beginning of the incubation, the medium was replaced with standard culture medium described above.

### In vitro mitogenic assay

The in vitro growth-inhibitory effects of TP53INP1 ASO on LNCaP cells were assessed using an assay based on the uptake and elution of crystal violet dye by the cells (Gleave M, Tolcher A, Miyake H, et al. "Progression to androgen independence is delayed by adjuvant treatment with antisense Bcl-2 oligodeoxynucleotides after castration in the LNCaP prostate tumor model". Clin Cancer Res 1999;5:2891-2898, **[28]**). One day after ASO treatment, cells were fixed in 1% glutaraldehyde (Sigma), stained with 0.5% crystal violet (Sigma), and eluted with 500 µl of Sorensen's solution (9 mg of trisodium citrate in 305 ml of distilled H20, 195 ml of 0.1 N HCl, and 500 ml of 90% ethanol). Absorbance of each well was measured by a Titertek Multiskan TCC/340 (Flow Laboratories, McLean, VA) at 560nm. Each assay was done in quadruplicate.

### Flow cytometric analysis

Flow cytometric analysis of propidium iodide-stained nuclei was done as described previously (Rocchi P, So A, Kojima S, et al. Heat shock protein 27 increases after androgen ablation and plays a cytoprotective role in hormone-refractory prostate cancer. Cancer Res 2004;64:6595-6602, **[29]**). Briefly, one day after ASO treatment, cells were trypsinized and analyzed for relative DNA content on a dual laser flow cytometer (Beckman Coulter et Epics Elite, Beckman, Inc, Miami, FL). Each assay was done in triplicate.

### Measurement of Caspase 3/7 activity

One day after ASO treatment, a caspase 3/7 activity assay was used to measure the apoptotic effect of transfection. Caspase-3/7 activity was measured using the Apo-ONE Homogeneous Caspase-3/7 Assay Fluorometric Kit (Promega). Caspase-3 activity was measured by the cleavage of the fluorometric substrate Z-DEVD-R110 according to the instructions of the manufacturer (Promega). Next, Apo-ONE Homogeneous Caspase-3/7 Reagent (100µl) was added to each well of a black 96-well plate containing 100 µL of blank, control or treated cells culture media. Each experiment was performed in triplicate. The plate was covered with a plate sealer, incubated at room temperature for 30 minutes before measuring the fluorescence of each well.

### Statistical analysis

Statistical analysis for TMA results was performed using Statistica statistical softwares PASS software (NCSS, Kaysville UT). The correlations between TP53INP1 expression (Quick score levels e.i. percentage of positive area x mean optical density) and clinicopathological parameters (D'Amico score, Gleason grade, Gleason score, tumour stage, lymph node metastasis and biological relapse) were evaluated on univariate analysis using Tukey-Kramer and/ or Mann-Whitney U tests. A Cox multiple regression model was used for multivariate analysis. A value of p< 0.05 (*) was considered statistically significant.

For the in vitro experiments, results were expressed as the mean +/-SE. Statistical analysis was done with a one-way ANOVA followed by Fisher's protected least significant difference test. p≤0.05 was considered significant (*), p≤0.01 (**) and p≤0.001 (***).

### Results

TP53INP1 expression is increased in inflammatory prostate tissues, in high grade PIN lesions and in PC. In normal prostate tissues, TP53INP1 was only expressed in the cytoplasm of prostate basal cells. Interestingly, there was a neo-expression of TP53INP1 in the cytoplasm of prostate luminal cells in inflammatory prostate tissues, in high grade PIN lesions and in PC (Figure 2).

To confirm that TP53INP1 could be improved in the first step of inflammation-mediated prostatic carcinogenesis, we treated LNCaP cells in vitro with inflammatory cytokines.

Stimulation of LNCaP cells in vitro with inflammatory cytokines (TNFalpha and IL6) enhances the TP53INP1 mRNA levels. The effect of TNFalpha or IL6 on TP53INP1 expression in LNCaP cells was determined in vitro using quantitative Real-Time PCR. TP53INP1 mRNA levels were normalized to 18S levels, and the fold change between treated and untreated cells was determined. Relative to untreated cells, treatment of LNCaP cells with 40ng/ml TNFalpha or 10ng/ml IL6 increased the TP53INP1 mRNA levels by 2.25- or 2.41- fold respectively (***p≤0.001) after 6h and by 1.57- or 2.17- fold respectively (***p≤0.001) after 8h (Fig. 3).

In human PC, TP53INP1 overexpression correlates with poor prognostic factors and is an independent predictive factor of biological cancer relapse. On TMA, about 18% of cores were not interpretable (stroma, non tumoral area, unsticked cores). In few cases, the Gleason grade present on TMA cores was different than the one circled on HES paraffin section from the donor block. We decided to keep the real Gleason grade present on TMA cores for statistical analysis. By image analysis, Gleason grade 3 PC had a Quick Score (QS) =7.76, grade 4 PC had a QS=11.47 and grade 5 PC had a QS=8.43. Gleason Score 6 PC had a QS=7.39, score 7 PC had a QS=8.78, score 8 PC had a QS=10.2 and score 9 PC had a QS=9.10. QS was 8.39 in favourable D'Amico score, 8.85 in intermediate score and 9.42 in unfavourable score. QS was 8.72 in absence of lymph node invasion and 12.17 in case of lymph node invasion. QS was 8.14 in absence of biological relapse and 11.23 in case of biological relapse. QS was 8.85 in pT2 stage and 8.90 in pT3 stage. On univariate analysis, TP53INP1 expression was significantly higher in Gleason grade 4 tumours compared with Gleason grade 3 tumours (p=0.0000053) (Fig. 4a) and in Gleason score 8 tumours (p=0.04) compared with Gleason score 6 tumours (p=0.04) (not shown). However, this increase in TP53INP1 expression was not present in our TMA for Gleason grade 5 tumours probably due to a too low number of patients (5 out of 91). Furthermore, increase of TP53INP1 expression was significantly associated with unfavourable D'Amico score (p=0.01), lymph node invasion (p=0.04) and biological cancer relapse (p=0.000093) (Fig. 4b,c,d). Nevertheless, TP53INP1 expression was not significantly correlated to tumoral stage (p>0.05) (not shown). On multivariate analysis, TP53INP1 overexpression was an independent predictive factor of biological cancer relapse (p=0.001) (Fig. 4d). The other independent factor was Gleason score (p= 0.009) (not shown).

TP53INP1 is dose-dependently downregulated by antisense oligonucleotide (ASO) in vitro. LNCaP cells were treated with increasing concentrations of ASO in order to obtain the MED. Protein was extracted from transfected cells and TP53INP1 protein levels were analysed by Western blotting using 40µg of protein. TP53INP1 ASO treatment significantly reduced TP53INP1 protein expression up to 70% in a dose-dependent manner, whereas TP53INP1 protein expression was not significantly suppressed by scrambled ASO control (Fig. 5).

TP53INP1 ASO inhibits proliferation and induces apoptosis of LNCaP cells in vitro. To further study the function of TP53INP1 in prostate tissue, inhibition of TP53INP1 using TP53INP1 ASO was employed with the appropriate scrambled controls. TP53INP1 ASO treatment significantly reduced LNCaP cell growth by 33.3% (***p≤0.001) at a concentration of 70nM, 1 day post-treatment compared with scrambled ASO controls (Fig. 6a). The induction of apoptosis by TP53INP1 ASO was clearly shown using flow cytometry. After the same treatment schedule described above, the fraction of cells undergoing apoptosis (sub Go-G1 fraction) was significantly higher after treatment with TP53INP1 ASO at a concentration of 70nM compared with scrambled ASO controls (39 % versus 5.77 %) (**p≤0.01) (Fig. 6b). The induction of apoptosis by TP53INP1 was also shown using the measurement of the caspase 3/7 activity. The caspase 3/7 activity was significantly higher after treatment with TP53INP1 ASO at a concentration of 70nM compared with scrambled ASO controls (ratio = 2.94) (**p≤0.01) (Fig. 6c).

### References

[1] Gronberg H. Prostate cancer epidemiology. Lancet 2003;361:859-864.
[2] De Marzo AM, Marchi VL, Epstein JI, Nelson WG. Proliferative inflammatory atrophy of the prostate: implications for prostatic carcinogenesis. Am J Pathol 1999;155:1985-1992.
[3] Nelson WG, De Marzo AM, Isaacs WB. Prostate cancer. N Engl J Med 2003;349:366-381.
[4] Putzi MJ, De Marzo AM. Morphologic transitions between proliferative inflammatory atrophy and high-grade prostatic intraepithelial neoplasia. Urology 2000;56:828-832.
[5] Stapleton AM, Timme TL, Gousse AE, et al. Primary human prostate cancer cells harboring p53 mutations are clonally expanded in metastases. Clin Cancer Res 1997;3:1389-1397.
[6] Nowak J, Tomasini R, Mattei MG, et al. Assignment of tumor protein p53 induced nuclear protein 1 (TP53INP1) gene to human chromosome band 8q22 by in situ hybridization. Cytogenet Genome Res 2002;97:140E.
[7] Tomasini R, Samir AA, Carrier A, et al. TP53INP1s and homeodomain-interacting protein kinase-2 (HIPK2) are partners in regulating p53 activity. J Biol Chem 2003;278:37722-37729.
[8] Gironella M, Seux M, Xie MJ, et al. Tumor protein 53-induced nuclear protein 1 expression is repressed by miR-155, and its restoration inhibits pancreatic tumor development. Proc Natl Acad Sci U S A 2007;104:16170-16175.
[9] Tomasini R, Samir AA, Vaccaro MI, et al. Molecular and functional characterization of the stress-induced protein (SIP) gene and its two transcripts generated by alternative splicing. SIP induced by stress and promotes cell death. J Biol Chem 2001;276:44185-44192.
[10] Okamura S, Arakawa H, Tanaka T, et al. p53DINP1, a p53-inducible gene, regulates p53-dependent apoptosis. Mol Cell 2001;8:85-94.
[11] Tomasini R, Samir AA, Pebusque MJ, et al. P53-dependent expression of the stress-induced protein (SIP). Molecular and functional characterization of the stress-induced protein (SIP) gene and its two transcripts generated by alternative splicing. SIP induced by stress and promotes cell death. Eur J Cell Biol 2002;81:294-301.
[12] Cano CE, Gommeaux J, Pietri S, et al. Tumor protein 53-induced nuclear protein 1 is a major mediator of p53 antioxidant function. Cancer Res 2009;69:219-226.
[13] Jiang PH, Motoo Y, Garcia S, Iovanna JL, Pebusque MJ, Sawabu N. Down-expression of tumor protein p53-induced nuclear protein 1 in human gastric cancer. World J Gastroenterol 2006;12:691-696.
[14] Gommeaux J, Cano C, Garcia S, et al. Colitis and colitis-associated cancer are exacerbated in mice deficient for tumor protein 53-induced nuclear protein 1. Mol Cell Biol 2007;27:2215-2228.
[15] D'Amico AV, Whittington R, Malkowicz SB, et al. Predicting prostate specific antigen outcome preoperatively in the prostate specific antigen era. J Urol 2001;166:2185-2188.
[16] Stamey TA, McNeal JE, Freiha FS, Redwine E. Morphometric and clinical studies on 68 consecutive radical prostatectomies. J Urol 1988;139:1235-1241.
[17] Charpin C, Garcia S, Bonnier P, et al. Prognostic significance of Nm23/NDPK expression in breast carcinoma, assessed on 10-year follow-up by automated and quantitative immunocytochemical assays. J Pathol 1998;184:401-407.
[18] Charpin C, Garcia S, Bonnier P, et al. bcl-2 automated and quantitative immunocytochemical assays in breast carcinomas: correlation with 10-year follow-up. J Clin Oncol 1998;16:2025-2031.
[19] Charpin C, Garcia S, Bouvier C, et al. Automated and quantitative immunocytochemical assays of CD44v6 in breast carcinomas. Hum Pathol 1997;28:289-296.
[20] Charpin C, Garcia S, Bouvier C, et al. CD31/PECAM automated and quantitative immunocytochemical assays in breast carcinomas: correlation with patient follow-up. Am J Clin Pathol 1997;107:534-541.
[21] Dales JP, Garcia S, Carpentier S, et al. Long-term prognostic significance of neoangiogenesis in breast carcinomas: comparison of Tie-2/Tek, CD105, and CD31 immunocytochemical expression. Hum Pathol 2004;35:176-183.
[22] Dales JP, Garcia S, Meunier-Carpentier S, et al. Overexpression of hypoxia-inducible factor HIF-1alpha predicts early relapse in breast cancer: retrospective study in a series of 745 patients. Int J Cancer 2005;116:734-739.
[23] Charpin C, Vielh P, Duffaud F, et al. Quantitative immunocytochemical assays of P-glycoprotein in breast carcinomas: correlation to messenger RNA expression and to immunohistochemical prognostic indicators. J Natl Cancer Inst 1994;86:1539-1545.
[24] Altman DG, Lausen B, Sauerbrei W, Schumacher M. Dangers of using "optimal" cutpoints in the evaluation of prognostic factors. Je Natl Cancer Inst 1994;86:829-835.
[25] Charpin C, Giusiano S, Secq V, et al. Quantitative immunocytochemical profile to predict early outcome of disease in triple-negative breast carcinomas. Int J Oncol 2009;34:983-993.
[26] Charpin C, Secq V, Giusiano S, et al. A signature predictive of disease outcome in breast carcinomas, identified by quantitative immunocytochemical assays. Int J Cancer 2009;124:2124-2134.
[27] Garcia S, Dales JP, Charafe-Jauffret E, et al. Poor prognosis in breast carcinomas correlates with increased expression of targetable CD146 and c-Met and with proteomic basal-like phenotype. Hum Pathol 2007;38:830-841.
[28] Gleave M, Tolcher A, Miyake H, et al. Progression to androgen independence is delayed by adjuvant treatment with antisense Bcl-2 oligodeoxynucleotides after castration in the LNCaP prostate tumor model. Clin Cancer Res 1999;5:2891-2898.
[29] Rocchi P, So A, Kojima S, et al. Heat shock protein 27 increases after androgen ablation and plays a cytoprotective role in hormone-refractory prostate cancer. Cancer Res 2004;64:6595-6602.
[30] Tomasini et al Oncogene. 2005 Dec 8;24(55):8093-104.
[31] Lang, S.H. Br J Cancer. 2000 Feb;82(4):990-7.
[32] Wu HC, Hsieh JT, Gleave ME, Brown NM, Pathak S, Chung LW. Derivation of androgen independent human LNCaP prostatic cancer cell sublines : role of bone stromal cells. Int J Cancer 1994 ; 57 : 406-12.

## Claims

1. Compound inhibiting TP53INP1 activity, for use in treatment of prostate cancer.

2. Compound according to claim 1, wherein said inhibition is a total or partial inhibition of TP53INP1 activity.

3. Compound according to claim 1 or 2, which binds to TP53INP1 protein or to TP53INP1 transcript.

4. Compound according to claim 3, which binds to TP53iNP1β transcript isoform.

5. Compound according to claim 4, which binds specifically to TP53INP1β transcript isoform.

6. Compound according to any one of claims 4 or 5, which binds to the sequence SEQ ID NO: 11 on TP53INP1β transcript isoform.

7. Compound according to any one of the preceding claims, wherein said inhibition is a specific inhibition.

8. Compound according to any one of the preceding claims, which is chosen among the compounds comprising: anti-sense oligonucleotides, antibodies, peptides, chemical inhibitors.

9. Compound according to claim 8, in which anti-sense oligonucleotides are chosen among siRNA, miRNA, RNAi and shRNA oligonucleotides.

10. Compound according to claim 1, which has the sequence SEQ ID NO:5.

11. Method for in vitro diagnosing a prostate cancer, comprising the step of determining the level of activity of TP53INP1 in a patient-derived biological sample comprising a prostate cell, wherein an increase of said level of activity compared to the normal control level indicates that said subject suffers from prostate cancer.

12. Inhibitor of TP531NP1 activity having the sequence SEQ ID NO: 5.

13. Method of screening for compounds modulating the TP53INP1 activity comprising the steps of:
i. contacting a test compound with TP53INP1 protein, TP53INP1 transcript, or prostate cancer cell,
ii. detecting the binding activity between TP53INP1 protein or TP53INP1 transcript and the test compound, or the modulation of TP53INP1 activity in said cell,
iii. selecting a compound that binds to TP53INP1 protein or TP53INP1 transcript or that modulates the TP53INP1 activity in said cell.

14. Method according to claim 12, wherein said TP53INP1 transcript is TP53INP1β transcript isoform.

15. Pharmaceutical composition comprising an inhibitor as defined in claim 11, and a pharmaceutically acceptable vehicle.
